# EUROPÄISCHE PATENTSCHRIFT

(11) **EP 1 250 189 B1**
(45) Veröffentlichungstag und Bekanntmachung des Hinweises auf die Patenterteilung: **22.06.2005**
(21) Anmeldenummer: 01911495.8
(22) Anmeldetag: 17.01.2001
(51) Int. Cl.: B01J 20/32, B01J 20/20, C01B 31/08, B01J 21/18, A61K 9/14

(54) **VERFAHREN ZUR MODIFIZIERUNG DER OBERFLÄCHEN VON FEINPORÖSEN ADSORBENTIEN**
METHOD FOR MODIFYING THE SURFACES OF FINE-POROUS ADSORBENTS
PROCEDE DE MODIFICATION DES SURFACES D'ABSORBANTS A PORES MINCES

(30) Priorität: 17.01.2000 DE 10002080; 17.05.2000 DE 10024312
(43) Veröffentlichungstag der Anmeldung: 23.10.2002
(73) Patentinhaber: Fraunhofer-Gesellschaft zur Förderung der angewandten Forschung e.V., 80686 München (DE)
(72) Erfinder: GUDERIAN, Joachim, 44581 Castrop-Rauxel (DE); HEIL, Volker, 44147 Dortmund (DE); JELEN, Erich, 46459 Rees-Bienen (DE); WEBER, Andreas, 47058 Duisburg (DE)
(86) Internationale Anmeldenummer: PCT/EP2001/000503
(87) Internationale Veröffentlichungsnummer: WO 2001/052981

(56) Entgegenhaltungen:
- EP-A- 0 774 296
- EP-A- 0 934 919
- WO-A-00/72960
- WO-A-97/47382
- WO-A-99/25322
- PATENT ABSTRACTS OF JAPAN vol. 1997, no. 06, 30. Juni 1997 (1997-06-30) & JP 09 049829 A (AGENCY OF IND SCIENCE &TECHNOL), 18. Februar 1997 (1997-02-18)
- YARITA T ET AL: "In situ silylation of silica-based packings using supercritical fluid as reaction medium" JOURNAL OF CHROMATOGRAPHY A,NL,ELSEVIER SCIENCE, Bd. 724, Nr. 1, 16. Februar 1996 (1996-02-16), Seiten 373-377, XP004039583 ISSN: 0021-9673

## Beschreibung

Gegenstand der vorliegenden Erfindung ist ein Verfahren zur Herstellung von Arzneimitteln mittels Modifizierung der Oberflächen feinporöser Adsorbentien, bei dem sich die feinporösen Adsorbentien in einem überkritischen Fluid befinden und das Modifizierungsmittel in diesem Fluid gelöst und/oder dispergiert wird.

Je nach Größe der Poren unterscheidet man grobporöse (Porendurchmesser ≥ 20 µm) und feinporöse Substanzen (Porendurchmesser ≤ 20 µm). Nach IUPAC lassen sich die Feinporen weiter in Makroporen (Porendurchmesser > 50 nm), Mesoporen (Porendurchmesser 2-50 nm), Mikroporen (Porendurchmesser 0,4-2 nm) und Submikroporen (Porendurchmesser < 0,4 nm) einteilen. Stoffe, die Makro- und/oder Meso- und/oder Mikro- und/oder Submikroporen enthalten, werden auch als MMMS-poröse Stoffe bezeichnet.

Neben Erscheinungen der Kapillarität, Transportphänomen u.a. weisen MMMS-poröse Feststoffe eine stark vergrößerte Oberfläche auf, die umso größer ist, je höher die Anzahl und je geringer die Größe der vorhandenen Poren ist. Dabei wird im allgemeinen zwischen innerer und äußerer Oberfläche unterschieden. Soweit es sich nicht um Kapillarkondensation oder verwandte Effekte handelt, welche ihrem Wesen nach auf innere Oberflächen oder hinreichend enge Partikelzwischenräume beschränkt sind, treten die an inneren Oberflächen ablaufenden Vorgänge jedoch auch an äußeren Oberflächen auf.

Adsorbentien sind meist feste Stoffe, die aufgrund ihrer großen Oberfläche befähigt sind, bestimmte Substanzen aus gasförmigen oder flüssigen Mischungen an ihrer Grenzfläche anzureichern (zu adsorbieren). Je größer die Gesamtoberfläche des Adsorbens pro Volumeneinheit ist, desto höher ist seine Adsorptionsfähigkeit. Deshalb sind feinporöse Stoffe mit großen inneren Oberflächen im allgemeinen gute Adsorbentien. Die gebräuch-Auch eine Imprägnierung äußerer Substratoberflächen oder grobporöser Substanzen mittels überkritischer Fluide wird im Stand der Technik beschrieben (DE 42 02 320 A1, DE 44 04 839 A1). Die Anlagerung bzw. das Aufbringen von Stoffen an äußere Oberflächen oder in Grobporen folgt jedoch anderen physikalischen und chemischen Gesetzmäßigkeiten als diejenige in MMMS-Poren mit einem Porendurchmesser von ≤ 200 nm. Letztere wird durch ausschließlich in MMMS-Poren auftretende Phänomene derart geprägt, dass ein Anlagerungsverfahren an äußere Substratoberflächen oder grobporöse Substanzen nicht mit einem solchen von MMMS-poröse Adsorbentien vergleichbar ist.

Aufgabe der vorliegenden Erfindung ist es, die Nachteile des Standes der Technik zu überwinden und ein technisch und wirtschaftlich vorteilhaftes Verfahren zur Modifizierung der Oberflächen von feinporösen Adsorbentien mit einem Porendurchmesser ≤ 200 nm, insbesondere der inneren Oberflächen, bereitzustellen, bei dem die Poreninnenwände, insbesondere auch von Mikro- und Submikroporen, einer Modifizierung unterzogen werden. Insbesondere soll das Verfahren qualitativ hochwertige Oberflächenmodifizierungen, d.h. die Herstellung selektiver und katalytisch wirksamer Adsorbentien, ermöglichen.

Diese Aufgabe wird erfindungsgemäß durch die kennzeichnenden Merkmale des Hauptanspruchs gelöst. Spezielle Verfahrensmerkmale sind in den Unteransprüchen beschrieben.

Bei dem erfindungsgemäßen Verfahren zur Modifizierung der (inneren) Oberflächen wird ein feinporöses Adsorbens oder ein Gemisch mehrerer feinporöser Adsorbentien mit einem Porendurchmesser ≤ 200 nm verwendet. Nach der Eingangs erwähnten Klassifizierung handelt es sich folglich bei den verwendeten Substraten um MMMS-poröse Adsorbentien. Diese werden mit einem Fluid kontaktiert, welches sich unter überkritischen Bedingungen befindet und ein oder mehrere Modifizierungsmittel gelöst und/oder dispergiert enthält. Vorzugsweise lässt man die feinporösen Adsorbentien jedoch von dem überkritischen Fluid um- bzw. durchströmen, da dies zu besonders effektiven Resultaten bei der erfindungsgemäßen Modifizierung führt. Das Modifizierungsmittel wird entweder vor oder während des Kontaktierens in dem Fluid gelöst und/oder dispergiert.

Die Oberflächenmodifizierung erfolgt durch Aufbringung der gelösten und/oder dispergierten Modifizierungsmittel auf die Oberfläche der MMMS-porösen Substanzen, insbesondere auf die innere Oberfläche. Da - wie Eingangs erwähnt - die an inneren Oberflächen ablaufenden Vorgänge jedoch auch an äußeren Oberflächen ablaufen, ist hier unter der (inneren) Oberfläche feinporöser Adsorbentien sowohl die innere wie auch die Auch eine Imprägnierung äußerer Substratoberflächen oder grobporöser Substanzen mittels überkritischer Fluide wird im Stand der Technik beschrieben (DE 42 02 320 A1, DE 44 04 839 A1). Die Anlagerung bzw. das Aufbringen von Stoffen an äußere Oberflächen oder in Grobporen folgt jedoch anderen physikalischen und chemischen Gesetzmäßigkeiten als diejenige in MMMS-Poren mit einem Porendurchmesser von ≤ 200 nm. Letztere wird durch ausschließlich in MMMS-Poren auftretende Phänomene derart geprägt, dass ein Anlagerungsverfahren an äußere Substratoberflächen oder grobporöse Substanzen nicht mit einem solchen von MMMS-poröse Adsorbentien vergleichbar ist.

Die EP-A-0 934819 beschreibt ein Verfahren zur Herstellung eines mikroporösen Adsorbens durch Kontaktieren des Adsorbens mit einem überkritischen Fluid, welches Modifizierungsmittel gelöst enthält, in Gegenwart eines Reaktionsinitiators. Dabei entsteht ein mit dem Modifizierungsmittel beschichtetes Adsorbens.

Die WO 99 25322 A lehrt ein Verfahren, bei dem ein quervemetztes Polymer mit einem Arzeimittelwirkstoff, der in einem überkritischen Fluid gelöst ist , behandelt wird, so dass das Polymer mit dem Arzneimittelwirkstoff beladen wird.

Aufgabe der vorliegenden Erfindung ist es, die Nachteile des Standes der Technik zu überwinden und ein technisch und wirtschaftlich vorteilhaftes Verfahren zur Herstellung von Arzneimitteln mittels Modifizierung der Oberflächen von feinporösen Adsorbentien mit einem Porendurchmesser ≤ 200 nm, insbesondere der inneren Oberflächen, bereitzustellen, bei dem die Poreninnenwände, insbesondere auch von Mikro- und Submikroporen, einer Modifizierung unterzogen werden.

Diese Aufgabe wird erfindungsgemäß durch die kennzeichnenden Merkmale des Hauptanspruchs gelöst. Spezielle Verfahrensmerkmale sind in den Unteransprüchen beschrieben.

Bei dem erfindungsgemäßen Verfahren zur Modifizierung der (inneren) Oberflächen wird ein feinporöses Adsorbens oder ein Gemisch mehrerer feinporöser Adsorbentien mit einem Porendurchmesser ≤ 200 nm verwendet. Nach der Eingangs erwähnten Klassifizierung handelt es sich folglich bei den verwendeten Substraten um MMMS-poröse Adsorbentien. Diese werden mit einem Fluid kontaktiert, welches sich unter überkritischen Bedingungen befindet und ein oder mehrere Modifizierungsmittel gelöst und/oder dispergiert enthält. Vorzugsweise lässt man die feinporösen Adsorbentien jedoch von dem überkritischen Fluid um- bzw. durchströmen, da dies zu besonders effektiven Resultaten bei der erfindungsgemäßen Modifizierung führt. Das Modifizierungsmittel wird entweder vor oder während des Kontaktierens in dem Fluid gelöst und/oder dispergiert.

Die Oberflächenmodifizierung erfolgt durch Aufbringung der gelösten und/oder dispergierten Modifizierungsmittel auf die Oberfläche der MMMS-porösen Substanzen, insbesondere auf die innere Oberfläche. Da - wie Eingangs erwähnt - die an inneren Oberflächen ablaufenden Vorgänge jedoch auch an äußeren Oberflächen ablaufen, ist hier unter der (inneren) Oberfläche feinporöser Adsorbentien sowohl die innere wie auch die äußere Oberfläche zu verstehen. Bei der Aufbringung der Modifizierungsmittel können einerseits die im überkritischen Fluid gelösten Substanzen an der (inneren) Oberfläche des feinporösen Adsorbens angelagert d.h. adsorbiert werden. Die Adsorption kann dabei entweder durch Physisorption, z.B. durch elektrostatische oder van-der-Waals-Wechselwirkungen, oder durch Chemisorption erfolgen. Andererseits kann gleichzeitig oder alternativ mindestens ein Teil des im überkritischen Fluid befindlichen Modifizierungsmittels mit der (inneren) Oberfläche des feinporösen Adsorbens bzw. mit einer oder mehreren reaktiven Gruppen an dieser Oberfläche reagieren, wobei mindestens ein Reaktionsprodukt als Modifizierung auf dieser Oberfläche verbleibt.

Das erfindungsgemäße Verfahren eignet sich überraschenderweise hervorragend dazu, auch die inneren Oberflächen feinporöser Adsorbentien mit einem Porendurchmesser ≤ 200 nm, insbesondere von mikro- und submikroporösen Substanzen, zu modifizieren. Überkritische Fluide weisen gegenüber den nach dem Stand der Technik verwendeten flüssigen Lösungsmitteln eine deutlich geringere Viskosität und eine geringere Grenzflächenspannung auf. Hierdurch wird bei dem erfindungsgemäßen Verfahren eine gute Benetzung der erfindungsgemäßen Adsorbentien, insbesondere der mikro- und submikroporösen Oberfläche, sicher gestellt. Insbesondere ist dies auch ein wesentlicher Faktor für eine qualitativ hochwertige Modifizierung mikro- und submikroporöser Adsorbentien.

Nach erfolgter Modifizierung kann das überkritische Fluid sehr leicht bei niedrigen Temperaturen durch ggf. vollständige Druckentspannung in den gasförmigen Zustand überführt werden und hierdurch aus dem feinporösen Adsorbens wieder vollständig ausgetrieben werden. Durch die geringe Dichte und die geringe Oberflächenspannung im gasförmigen Zustand treten keine Kapillarkondensationseffekte auf, wie dies bei der thermischen Abtrennung von Lösungsmitteln nach den Stand der Technik der Fall ist. Somit kann das erfindungsgemäß verwendete Lösungsmittel relativ einfach und schonend wieder vollständig aus dem modifizierten Adsorbens entfernt werden.

Grundsätzlich ist jedes überkritische Fluid für das erfindungsgemäße Verfahren geeignet. Bevorzugt verwendet wird Kohlendioxid, Lachgas, niedere Alkane, insbesondere Propan und Butan, niedere Alkene, insbesondere Ethen, Methanol oder eine Mischung derselben.

Das erfindungsgemäße Verfahren wird in der Regel bei Drücken zwischen 5,0 und 40.0 MPa und Temperaturen zwischen 15 und 250°C durchgeführt. Die genauen Druck- und Temperatur-Werte sind von dem verwendeten überkritischen Fluid abhängig und können von Fachmann aufgrund seines allgemeinen Fachwissen geeignet gewählt werden.

Allgemein lässt sich sagen, dass für das erfindungsgemäße Verfahren die überkritischen Fluide insbesondere in der Nähe ihres kritischen Punktes verwendet werden.

Durch Variation der äußeren Parameter Druck und Temperatur können die physikalischen Eigenschaften überkritischer Fluide, die unmittelbaren Einfluss auf die Löslichkeit des gelösten Modifizierungsmittels haben, verändert werden. Dies hat auch Auswirkungen auf den Abscheidungsprozess der gelösten Modifizierungsmittel auf den Oberflächen der Adsorbentien, so dass über eine Variation dieser Parameter sowohl die Menge des adsorbierten Modifizierungsmittels wie auch die Größe der modifizierten Poren gezielt gesteuert werden kann. So lässt sich beispielsweise durch eine Variation des Drucks zwischen dem ein- und dem vierfachen kritischen Druck des Fluids die Löslichkeit der erfindungsgemäß geeigneten Modifizierungsmittel derart einstellen, dass im Sättiaungszustand zwischen etwa 0.1 und 10 Gew.-% im überkritischen Fluid gelöst sind. Die Temperaturabhängigkeit im Hinblick auf die Löslichkeit ist komplizierter. Sie hängt in erster Linie vom Phasengleichgewicht zwischen dem Modifizierungsmittel und dem überkritischen Fluid ab. In erster Näherung nimmt mit ansteigender Temperatur die Dichte des Fluids und damit sein Lösungsvermögen ab.

Durch geeignete Führung der beiden Parameter Druck und Temperatur lässt sich die Abscheidung des Modifizierungsmittels in den Poren des feinporösen Adsorbens vorteilhafterweise soweit steuern, dass weitergehender Einfluss auf die Morphologie der abgeschiedenen Schichten genommen werden kann. Beispielsweise führt eine schnelle Abscheidung durch schnelle Druckentspannung zu amorphen Schichten, während bei entsprechend langsamerer Druckentspannung kristalline Schichten entstehen können.

Die Temperatur kann auch variiert werden, um zusätzliche Effekte, wie z.B. die Temperaturabhängigkeit der Bildung verschiedener fester Phasen auf der (inneren) Oberfläche auszunutzen. Durch das Unter- oder Überschreiten von Glasumwandlungstemperaturen und anderen Phasenübergangstemperaturen kann daher ein geeignetes Modifizierungsmittel in verschiedenen festen Morphologien auf die (innere) Oberfläche des feinporösen Adsorbens aufgebracht werden. Beispielsweise verfestigen sich als Modifizierungsmittel einsetzbare Fettsäuren bei niedrigeren Temperaturen (abhängig von der konkret verwendeten Fettsäure) kristallin, während sie bei höheren Temperaturen flüssig kristalline Phasen bilden. Bei den Verfahren nach dem Stand der Technik können derartige Effekte nicht ausgenutzt werden.

Als Modifizierungsmittel kann jede Substanz verwendet werden, die dazu geeignet ist, durch die Modifizierung der inneren Oberfläche eines feinporösen Adsorbens die Eigenschaften des Adsorbens zu beeinflussen. Beispiele für erfindungsgemäße Modifizierungsmittel sind organische Verbindungen, metallorganische Verbindungen, anorganische Verbindungen oder Gemische dieser Substanzen. Auch Mikroemulsionen wasserlöslicher Salze in Micellen geeigneter Tenside können als Modifizierungsmittel eingesetzt werden. Daneben können auch alle Substanzen, die im Stand der Technik zur Modifizierung der Oberflächen feinporöser Adsorbentien bekannt sind, verwendet werden.

Ohne Einschränkung der Allgemeinheit sind konkrete organische Modifizierungsmittel beispielsweise Benzol, Glycerin, Fettsäuren, Benzoesäure, Polyethylenglykole, Squalan, Paraffine, Octadecan. Diese Substanzen sind insbesondere für die Herstellung von Molekularsieben geeignet. Neben anderen können als metallorganische Modifizierungsmittel z.B. Titan-, Zirkonium-, Vanadium-, Yttrium-, Kupfer- Molybdän-ethoxylate oder -isopropylate, oder Koordinationsverbindungen wie z.B. Ferrocen, Metallocene oder Übergangsmetallkomplexe mit Tetramethylheptandionen oder Diisopropyldithiocarbamaten verwendet werden. Dies führt in der Regel zu katalytisch aktiven Adsorbentien. Die auf die (innere) Oberfläche der Adsorbentien aufgebrachten metallhaltigen Verbindungen können im Rahmen einer Nachbehandlung reduziert werden, so dass die (innere) Oberfläche im Endeffekt mit Metallen modifiziert ist.

In der Regel werden 0,1 bis 10 Gew.-% Modifizierungsmittel bezogen auf die Lösung bzw. Dispersion des überkritischen Fluids eingesetzt. Hierbei muss die Beladung des überkritischen Fluids mit dem Modifizierungsmittel auf die äußeren Bedingungen wie Druck und Temperatur abgestimmt werden. Der Fachmann ist jedoch in der Lage die geeigneten Parameter aufgrund seines Fachwissens zu wählen.

Zur Durchführung des erfindungsgemäßen Verfahrens ist grundsätzlich jedes feinporöse Adsorbens geeignet. Dabei können die verwendeten Adsorbentien jeweils ausschließlich oder vorwiegend Makro-, Meso-, Mikro- oder Submikroporen oder ein Gemisch dieser Poren aufweisen. Bevorzugt werden Kohlenstoff haltige Adsorbentien. Zeolithe. Kieselgele, Aluminiumoxide oder Gemische derselben modifiziert. Als Kohlenstoff haltiges Adsorbens sind dabei besonders bevorzugt Aktivkohle, Aktivkoks oder Kohlenstoffmolekularsiebe.

Bevorzugt werden MMMS-poröse Adsorbentien modifiziert, bei denen ≥ 30 Vol.-% des Porenvolumens auf Poren mit Porendurchmesser ≤ 50 nm entfällt. Besonders bevorzugt werden MMMS-poröse Adsorbentien modifiziert, bei denen ≥ 30 Vol.-% des Porenvolumens auf Poren mit Porendurchmesser ≤ 2 nm entfällt.

Das MMMS-poröse Adsorbens kann auch von Adsorbaten befreit oder mit adsorbierten Adsorbaten vorliegen. Im Fall von Wasser als Adsorbat bedeutet dies beispielsweise, dass entweder ein getrocknetes oder ein ungetrocknetes Adsorbens verwendet wird. Letzteres wird z.B. bevorzugt, wenn die Modifizierung unter gleichzeitiger Hydrolyse des Modifizierungsmittels erfolgen soll. Bei den Verfahren nach dem Stand der Technik ist eine Modifizierung unter gleichzeitiger hydrolytischer Reaktion des Modifizierungsmittels - wenn überhaupt - nur bedingt möglich.

Ferner besteht die Möglichkeit, dass als Edukt feinporöse Adsorbentien ohne oder mit bereits erfolgter Oberflächenmodifizierung verwendet werden, wobei im letzteren Fall die Modifizierung erfindungsgemäß oder auf andere Weise erfolgt sein kann.

Neben einer Verwendung als Endprodukt kann das erfindungsgemäß modifizierte Adsorbens als Zwischenprodukt zur weiteren physikalischen und/oder chemischen Behandlung und/oder zur weiteren Modifizierung nach den erfindungsgemäßen Verfahren oder einem anderen Modifizierungsverfahren nach dem Stand der Technik verwendet werden.

In einer besonderen Ausführungsform erhält das feinporöse Adsorbens durch die erfindungsgemäße Oberflächenmodifizierung der inneren Oberfläche katalytische Eigenschaften.

Besonders vorteilhaft kann das erfindungsgemäße Verfahren zur Herstellung eines Molekularsiebes verwendet werden. Hierbei wird die innere Oberfläche z.B. eines Kohlenstoff haltigen feinporösen Adsorbens, insbesondere Aktivkohle oder Aktivkoks, mit Substanzen modifiziert, die bei einer Pyrolyse Kohlenstoff bilden können. Ohne Einschränkung der Allgemeinheit sind Beispiele für bei der Pyrolyse Kohlenstoff abspaltende Substanzen Benzol, Benzoesäure, Fettsäuren, Glycerin und/oder Polyethylenglykole. Die verwendeten Modifizierungsmittel können je nach ihrer Molekülgröße nur in die entsprechend größeren Poren eindringen und diese ggf. verschließen, während kleinere Poren unmodifiziert bleiben. In einer der Modifizierung folgenden thermischen Behandlung des modifizierten Adsorbens wird insbesondere der Kohlenstoff aus den eingebrachten Modifizierungsmitteln abgespalten und verbleibt in den Poren, während gasförmige und flüchtige Reaktionsprodukte aus dem Porensystem ausgetrieben werden.

Die in die Poren eingebrachten Modifizierungsmittel führen dazu, dass aus ursprünglich größeren Poren kleinere Poren entstehen, so dass es insgesamt zu einer Erhöhung der Anzahl an kleineren Poren kommt. Durch das erfindungsgemäße Verfahren ist es somit möglich, Kohlenstoffmolekularsiebe mit einer definierten und engen Porengrößenverteilung herzustellen.

In einer bevorzugten Ausführungsform können die Eigenschaften des erfindungsgemäß hergestellten Kohlenstoffmolekularsiebes gezielt verändert werden. Hierbei wird im Anschluss an die thermische Behandlung das Kohlenstoffmolekularsieb analog zum Stand der Technik einer anschließenden Aktivierung unterzogen, wobei die verengten Poren um einen definierten Wert aufgeweitet werden (Carbon, 33 (1995), Nr.12, 1717-1725). Besonders bevorzugt erfolgt eine chemische oder eine Dampfaktivierung.

In weiteren einer besonderen Ausführungsform kann das erfindungsgemäße Verfahren zur Herstellung eines Arzneimittels verwendet werden. Hierbei wird innere Oberfläche eines Kohlenstoff haltigen Adsorbens, insbesondere Aktivkohle und/oder CMS (Kohlenstoffmolekularsieb/e), mit einem oder mehreren Modifizierungsmitteln, insbesondere Arzneimittelwirkstoffen, oberflächenmodifiziert. Derartige, an Aktivkohle oder CMS gebundene Pharmazeutika sind vorteilhafterweise dazu geeignet, einen konstanten Wirkstoffspiegel im Körper zu erzielen, indem sie die an den feinporösen Kohlenstoff gebundenen Arzneimittelwirkstoffe kontrolliert abgeben (controlled release). Dabei ist die medizinische Unbedenklichkeit von Aktivkohlepräparaten durch die seit vielen Jahren praktizierte Verwendung von Kohle bei diarrhöischen Erkrankungen zur Adsorption von Toxinen u.ä. gegeben. Vorteilhaft ist weiterhin, dass die Modifizierung mit einem überkritischen Fluid den Wirkstoff sehr schonend in die Trägersubstanz, das Adsorbens, einbringt und dass die Löslichkeit vieler Arzneistoffe in überkritischen Fluiden gut ist. Ferner sind aufgrund des einfachen Modifizierungsschrittes mit dem erfindungsgemäßen Verfahren hergestellte Arzneimittel deutlich kostengünstiger und wirtschaftlicher als beispielsweise nach dem Stand der Technik aufwendig hergestellte Controlles-Release-Arzneimittel auf Microcomposite-Basis.

Vorteilhafterweise besteht bei dem erfindungsgemäßen Verfahrens die Möglichkeit, zunächst einen oder mehrere Substanzen an einem feinporösen Adsorbens zu adsorbieren und diese dann gemäß dem erfindungsgemäßen Verfahren mit einem Modifizierungsmittel reagieren zu lassen. Hierdurch können ein oder mehrere Reaktionsprodukte zur Modifizierung der inneren Oberfläche angelagert werden, während andere Reaktionsprodukte gegebenenfalls in dem überkritischen Fluid gelöst werden.

Überraschenderweise ist es möglich mit dem erfindungsgemäßen Verfahren Modifizierungsmittel in feinporöse Adsorbentien, insbesondere in Mikro- und Submikroporen, gezielt einzubringen. Auch können mit dem erfindungsgemäßen Verfahren qualitativ hochwertige Oberflächenmodifizierungen, d.h. die Herstellung selektiver und katalytisch wirksamer Adsorbentien, vorgenommen werden. Das erfindungsgemäße Verfahren stellt im Vergleich zum Stand der Technik ein technisch und wirtschaftlich vorteilhaftes Verfahren zur Modifizierung der inneren Oberflächen von feinporösen Adsorbentien dar.

Ohne Einschränkung der Allgemeinheit wird das erfindungsgemäße Verfahren anhand einiger Ausführungsbeispiele näher erläutert.

### 1. Modifizierung einer Aktivkohle mit Zirkonium(IV)oxid

Es werden 0,15 g Körner einer ungetrockneten, nicht modifizierte Formaktivkohle für die Gasreinigung eingewogen und mit einer Lösung von 0,2 g Zirkoniumisopropylat in 4,5 ml überkritischem Kohlendioxid (SCCO₂) bei einer Temperatur von 40°C und einem Druck von 150 bar ca. 30 Minuten kontaktiert. Erhalten werden dabei braun-gräuliche Aktivkohlepellets, die sowohl auf der Außenseite wie auch im Korninneren weiße Partikel enthielten, da das Zirkoniumisopropylat sehr schnell hydrolysiert und unter den genannten Bedingungen weißes, hochreines Zirkonium(IV)oxid mit einer großen Oberfläche liefert. Das zur Hydrolyse benötigte Wasser stammt dabei aus der ungetrockneten Aktivkohle.
Die Gewichtszunahme der Formaktivkohle durch die Modifizierung beträgt ca. 20 %.

### 2. Modifizierung einer Aktivkohle mit Palmitinsäure

Es werden 0,5 g Körner einer ungetrockneten, nicht modifizierten Formaktivkohle für die Gasreinigung eingewogen und mit einer Lösung von 0,14 g Palmitinsäure und 0,05 g 2-Propanol in 4,5 ml überkritischen Kohlendioxid bei einer Temperatur von 40°C und einem Druck von 120 bar 20 Minuten lang kontaktiert. Anschließend wird der Druck langsam (2 bar/min) entspannt, um das Kohlendioxid von der Kohle zu desorbieren, wobei die adsorbierte Palmitinsäure und ein Teil des 2-Propanols auf der Aktivkohle zurückbleiben.
Zur weiteren Modifizierung der inneren Oberfläche wird die modifizierte Aktivkohle bei 900°C 60 Minuten thermisch behandelt.

### 3. Modifizierung einer Aktivkohle mit Octadecan

Es werden 0,5 g Körner einer ungetrockneten, nicht modifizierten Gasreinigungs-Formaktivkohle eingewogen und mit einer Lösung von 0,25 g Octadecan in 4,5 ml überkritischen Kohlendioxid bei einer Temperatur von 50°C und einem Druck von 200 bar 30 Minuten lang kontaktiert. Anschließend wird der Druck langsam (2 bar/min) entspannt, um das Kohlendioxid von der Kohle zu desorbieren, wobei das adsorbierte Octadecan auf der Aktivkohle zurückbleibt.
Die erhaltene Aktivkohle eignet sich insbesondere für spezielle Adsorptions- und Chromatographieprozesse.

## Patentansprüche

1. Verfahren zur Herstellung eines Arzneimittels,
**dadurch gekennzeichnet, dass** ein Kohlenstoff-haltiges feinporöses Adsorbens, das einen Porendurchmesser ≤ 200 nm aufweist, mit einem oder mehreren Arzneimittetwirkstoffen oberflächenmodifiziert wird,
indem das Adsorbens mit einem überkritischen Fluid kontaktiert wird, welches einen oder mehrere Arzneimittelwirkstoffe gelöst und/oder dispergiert enthält.

2. Verfahren nach Anspruch 1,
**dadurch gekennzeichnet, dass** als Kohlenstoff-haltiges feinporöses Adsorbens Aktivkohle und/oder ein Kohlenstoffmolekularsieb modifiziert wird.

3. Verfahren nach mindestens einem der Ansprüche 1 oder 2,
**dadurch gekennzeichnet, dass** ein feinporöses Adsorbens verwendet wird, bei dem ≥ 30 Vol.-% des Porenvolumens auf Poren mit einem Porendurchmesser von ≤ 50 nm, insbesondere ≤ 2 nm, entfällt.

4. Verfahren nach mindestens einem der Ansprüche 1 bis 3,
**dadurch gekennzeichnet, dass** als überkritisches Fluid Kohlendioxid, Lachgas, niedere Alkane, insbesondere Propan und/oder Butan, und/oder niedere Alkene, insbesondere Ethen, und/oder Methanol verwendet werden.

5. Verfahren nach mindestens einem der Ansprüche 1 bis 4,
**dadurch gekennzeichnet, dass** als Arzneimittelwirkstoff eine organische Verbindung, eine metallorganische Verbindung und/oder eine anorganische Verbindung verwendet wird.

6. Verfahren nach mindestens einem der Ansprüche 1 bis 5,
**dadurch gekennzeichnet, dass** für die Oberflächenmodifizierung ein bereits oberflächenmodifiziertes feinporöses Adsorbens verwendet wird.

7. Verfahren nach mindestens einem der Ansprüche 1 bis 6,
**dadurch gekennzeichnet, dass** das feinporöse Adsorbens nach der Oberflächenmodifizierung physikalisch und/oder chemisch behandelt und/oder weiter oberflächenmodifiziert wird.

## Claims

1. A method for preparation of a medicinal product, **characterized in that** a carbon-containing fine-porous adsorbent, that has a diameter of ≤ 200 nm, has its surface modified with one or a plurality of active medicinal ingredients, in which the adsorbent is contacted with a supercritical fluid, which contains one or a plurality of dissolved and/or dispersed active medicinal ingredients.

2. The method according to Claim 1, **characterized in that** as carbon-containing fine-porous adsorbent, activated carbon and/or a carbon molecular sieve is modified.

3. The method according to at least one of Claims 1 or 2, **characterized in that** a fine-porous adsorbent is used in which ≥ 30 vol% of the pore volume exhibits pores having a pore diameter of ≤ 50 nm, especially ≤ 2 nm.

4. The method according to at least one of Claims 1 through 3, **characterized in that** as supercritical fluid, carbon dioxide, nitrous oxide, lower alkanes, especially propane and/or butane, and/or lower alkenes, especially ethylene, and/or methanol are used...

5. The method according to at least one of Claims 1 through 4, **characterized in that** as active medicinal ingredient, an organic compound, an organometallic compound and/or an inorganic compound is used.

6. The method according to at least one of Claims 1 through 5, **characterized in that** for the surface modification an already surface-modified fine-porous adsorbent is used.

7. The method according to at least one of Claims 1 through 6, **characterized in that** after the surface modification, the fine-porous adsorbent is physically and/or chemically treated and/or is further surface-modified.

## Revendications

1. Procédé de fabrication d'un médicament,
**caractérisé en ce qu'**
un adsorbant à pores minces contenant du carbone, et qui présente un diamètre de pores ≤ 200 nm, est modifié en surface avec un ou plusieurs principes actifs médicamenteux, en mettant l'adsorbant en contact avec un liquide supercritique qui contient un ou plusieurs principes actifs médicamenteux en solution et/ou dispersés.

2. Procédé selon la revendication 1,
**caractérisé en ce que**
comme adsorbant à pores minces contenant du carbone, on modifie du charbon actif et/ou un tamis moléculaire de carbone.

3. Procédé selon l'une quelconque des revendications 1 ou 2,
**caractérisé en ce qu'**
on utilise un adsorbant à pores minces dans lequel on supprime ≥ 30 % (V/V) du volume des pores constitué de pores dont le diamètre est ≤ 50 nm, de préférence ≤ 2 nm.

4. Procédé selon l'une quelconque des revendications 1 à 3,
**caractérisé en ce que**
comme liquide supercritique on utilise du dioxyde de carbone, du protoxyde d'azote, des alcanes à faible nombre d'atomes de carbone, en particulier le propane et/ou le butane, et/ou des alcènes à faible nombre d'atomes de carbone, en particulier l'éthylène et/ou le méthanol.

5. Procédé selon l'une quelconque des revendications 1 à 4,
**caractérisé en ce que**
comme principe actif médicamenteux on utilise un composé organique, un composé mêtallorganique et/ou un composé anorganique.

6. Procédé selon l'une quelconque des revendications 1 à 5,
**caractérisé en ce que**
pour la modification en surface on utilise un adsorbant à pores minces déjà modifié en surface.

7. Procédé selon l'une quelconque des revendications 1 à 6,
**caractérisé en ce qu'**
après la modification en surface, l'adsorbant à pores minces est traité physiquement et/ou chimiquement, et/ou davantage modifié en surface.
